# EUROPEAN PATENT APPLICATION

(11) **EP 4 218 714 A1**
(43) Date of publication of application: **02.08.2023**
(21) Application number: 21872128.0
(22) Date of filing: 03.09.2021
(51) Int. Cl.: A61K 8/73, A61K 8/06, A61K 8/92, A61Q 19/00

(54) **EMULSIFIED COMPOSITION FOR SKIN**

(30) Priority: 28.09.2020 JP 2020161951
(71) Applicant: House Wellness Foods Corporation, Itami-shi Hyogo, 644-0011 (JP); House Foods Corporation, Higashi-Osaka-shi, Osaka 577-8520 (JP)
(72) Inventor: TOMOTAKE Muneaki, Itami-shi, Hyogo 644-0011 (JP); ISHIDA Ryosuke, Itami-shi, Hyogo 644-0011 (JP); HIRAYAMA Yoshitake, Itami-shi, Hyogo 644-0011 (JP); SASAKO Hiroshi, Higashi-osaka-city, Osaka 5778520 (JP)
(74) Representative: Jones, Nicholas Andrew
(86) International application number: PCT/JP2021/032376
(87) International publication number: WO 2022/064997

(57) **Abstract**

An object of the present invention is to provide an emulsified composition containing fat and oil, water, and cyclodextrin, which has high emulsion stability and a good feeling of use when applied to the skin. Provided is an emulsified composition for skin containing water, fat and oil, cyclodextrin, and a prescribed water-soluble gelling agent.

## Description

### Technical Field

The present invention relates to an emulsified composition for skin containing water, fat and oil, cyclodextrin, and a prescribed water-soluble gelling agent.

### Background Art

Emulsified compositions containing fat and oil, water, and cyclodextrin are widely used in various fields of, for example, pharmaceuticals, cosmetics, and foods and drinks.

Patent Literature 1 discloses an emulsified cosmetic obtained by mixing an aqueous raw material in which a water-soluble polymer compound such as pectin, tragacanth gum, gelatin, casein, sodium alginate, hydroxypropyl cellulose, hydroxyethyl cellulose, hydroxypropylmethyl cellulose, methyl cellulose, sodium polyacrylate, or carboxyvinyl polymer is dissolved with an oily raw material for a cosmetic, adding cyclodextrin thereto, and emulsifying the mixture.

Patent Literature 2 discloses an emulsified composition characterized by containing α-cyclodextrin, polyoxyethylene glycol, and an oily component, and also describes a skin topical preparation characterized by containing the emulsified composition.

Non Patent Literature 1 describes that emulsion stability increased when xanthan gum and tragacanth gum were each added as an emulsion stabilizer to an oil-drops-in-water type emulsion obtained by emulsifying a sample of soybean oil and an aqueous cyclodextrin solution at a volume ratio of 1:1.

### Citation List

### Patent Literature

Patent Literature 1: Japanese Patent Publication No. 61-038166
Patent Literature 2: Japanese Patent Laid-Open No. 09-124437

### Non Patent Literature

Non Patent Literature 1: Nippon Shokuhin Kogyo Gakkaishi, Vol. 38, No. 1, 16-20 (1991)

### Summary of Invention

### Technical Problem

The present inventors have found that when a conventionally known emulsified composition containing fat and oil, water, and cyclodextrin is used for application to the skin, the oil and water may be easily separated from each other because emulsion stability of the emulsified composition is not sufficient and that the emulsified composition may fail to provide a good feeling of use due to stickiness, sliminess, or the like when applied to the skin.

Therefore, an object of the present invention is to provide an emulsified composition containing fat and oil, water, and cyclodextrin, which has high emulsion stability, is used for application to the skin, and has a good feeling of use when applied to the skin.

### Solution to Problem

As a result of intensive studies to solve the problem, the present inventors have found that an emulsified composition suitable for application to the skin, which has high emulsion stability and a good feeling of use when applied to the skin, can be obtained by blending at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum together with fat and oil, water, and cyclodextrin, and have completed the present invention. In particular, when the emulsified composition having high emulsion stability is spread on the skin, the feeling of use is unprecedented. The emulsified composition has a good feeling of use without oiliness (stickiness or sliminess) even immediately after application and simultaneously provides a smooth feeling as if a powder is applied and a moist feeling as if a cream is applied when washed with water or the like after application. The emulsified composition is not limited to any particular form, and it has been confirmed that the emulsified composition can provide the same feeling of use even in a dried form (e.g., powder) when spread on the skin.

The present invention is based on these new findings, and the following inventions are included.
[1] An emulsified composition for skin, containing water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum.
[2] The emulsified composition according to [1], wherein the fat and oil is an edible vegetable oil.
[3] The emulsified composition according to [1] or [2], which is a hand cream.
[4] A method for producing an emulsified composition for skin, including mixing water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum together.
[5] The method according to [4], wherein the fat and oil is an edible vegetable oil.
[6] The method according to [4] or [5], wherein the emulsified composition for skin is a hand cream.

The present specification encompasses the contents described in, for example, the specifications of Japanese Patent Application No. 2020-161951, filed on Sep. 28, 2020, which is the basis of the priority of the present application.

All publications, patents, and patent applications cited herein are hereby incorporated by reference in their entirety.

### Advantageous Effects of Invention

According to the present invention, it is possible to provide an emulsified composition containing fat and oil, water, and cyclodextrin, which has high emulsion stability, is used for application to the skin, and has a good feeling of use when applied to the skin.

### Description of Embodiments

The present invention relates to an emulsified composition for skin containing water, fat and oil, cyclodextrin, and a prescribed water-soluble gelling agent.

As the "fat and oil" in the present invention, fat and oil commonly used in an emulsified composition for skin is available, and both polar and non-polar fats or oils may be used. Examples of such fat and oil include, but are not limited to, plant-derived fat and oil (e.g., canola oil, refined rapeseed oil, soybean oil, corn oil, cottonseed oil, peanut oil, sesame oil, rice oil, rice bran oil, camellia oil, safflower oil, olive oil, linseed oil, Japanese basil oil, perilla oil, sunflower oil, palm oil, tea oil, palm oil, avocado oil, Kukui nut oil, grapeseed oil, cocoa butter, coconut oil, wheatgerm oil, almond oil, evening primrose oil, castor oil, hazelnut oil, macadamia nut oil, rosehip oil, grape oil, cacao oil, jojoba oil, palm kernel oil, Japan wax, candelilla wax, and carnauba wax), synthetic ester oils such as paraffin, microcrystalline wax, ceresin, vaseline, ozokerite, isostearyl alcohol, caprylic alcohol, lauryl alcohol, stearyl alcohol, 2-octadecyl alcohol, myristyl alcohol, cetyl alcohol, phytosterol, cholesterol, stearic acid, isostearic acid, capric acid, lanolin acid, lauric acid, myristic acid, palmitic acid, behenic acid, linolic acid, linolenic acid, glyceryl monostearate, glyceryl monopalmitate, glyceryl monobehenate, glyceryl monomyristate, glyceryl monolaurate, glyceryl monolanolate, glyceryl monolinolate, glyceryl monolinolenate, glyceryl monooleate, glycerol triisostearate, isopropyl myristate, glycerol tri-2-heptylundecanoate, glycerol tri-2-ethylhexanoate, 2-heptylundecyl palmitate, di-2-heptylundecyl adipate, cetyl isooctanoate, trimethylolpropane-2-trimethylolheptylundecanoate, propane-2-ethylhexanoate, pentaerythritol-2-heptylundecanoate, pentaerythritol-2-ethylhexanoate, cholesterol isostearate, diethyl phthalate, and dibutyl phthalate, animal-derived fats or oils such as beef tallow, lard, lanolin, squalene, squalane, beeswax, and spermaceti, and silicone oil. In the present invention, the fat and oil is preferably animal-derived or plant-derived fat and oil with high safety and particularly preferably an edible vegetable oil that has been eaten and is highly safe. The fat and oil may be used alone or in combination with different fat and oil.

The emulsified composition for skin of the present invention may contain fat and oil in any amount, for example, in an amount of 5 wt% or more, or 10 wt% or more. The upper limit thereof is not particularly limited, but may be, for example, 50 wt% or less, 40 wt% or less, 30 wt% or less, or 20 wt% or less. The emulsified composition for skin of the present invention may contain the fat and oil in an amount appropriately selected from the range of, for example, 5 wt% to 50 wt%, preferably 10 wt% to 30 wt%, and more preferably 10 wt% to 20 wt%. If the amount of fat and oil is less than 5 wt%, emulsification may be insufficient, or the emulsified composition may give a creaky feeling when applied to the skin. On the other hand, if the amount of fat and oil is more than 50 wt%, the emulsified composition may give strong oiliness such as stickiness or sliminess when applied to the skin. In either case, the composition may not provide a favorable feeling of use when applied to the skin.

In the emulsified composition for skin of the present invention, water may be contained in any amount capable of emulsifying fat and oil to form an oil-in-water emulsion together with cyclodextrin and a prescribed water-soluble gelling agent. For example, the emulsified composition for skin of the present invention may contain water in an amount of 15 wt% or more, 20 wt% or more, 30 wt% or more, 40 wt% or more, 45 wt% or more, 50 wt% or more, 60 wt% or more, or 70 wt% or more. The upper limit thereof is not particularly limited, but may be, for example, 90 wt% or less, or 85 wt% or less. The emulsified composition for skin of the present invention may contain water in an amount appropriately selected from the range of, for example, 15 wt% to 90 wt%, preferably 45 wt% to 90 wt%, and more preferably 70 wt% to 85 wt%. The amount of water contained in the emulsified composition for skin of the present invention is preferably more than that of fat and oil in volume ratio. For example, the content of fat and oil and water can be 1: greater than 1 (fat and oil: water) in volume ratio, such as 1:2 or more, 1:3 or more, 1:4 or more, 1:5 or more, or 1:6 or more. The upper limit of the amount of water is not particularly limited, but can be 1:10 or less, and preferably 1:8 or less. Through adjustment of the amounts of water and fat and oil contained in the emulsified composition for skin of the present invention within the above ranges, the emulsified composition may have suppressed oiliness such as stickiness and sliminess when applied to the skin and provide a favorable feeling of use.

"Cyclodextrin" means a cyclic non-reducing maltooligosaccharide having glucose as a constituent unit, and examples thereof include α-cyclodextrin having six glucose units, β-cyclodextrin having seven glucose units, and γ-cyclodextrin having eight glucose units. In the present invention, α-, β-, and γ-cyclodextrins, cyclodextrin derivatives, and any combination thereof can be used. Examples of the cyclodextrin derivatives include, but are not limited to, ethyl cyclodextrin, methyl cyclodextrin, hydroxyethyl cyclodextrin, hydroxypropyl cyclodextrin, methylamino cyclodextrin, amino cyclodextrin, carboxyethyl cyclodextrin, carboxymethyl cyclodextrin, sulfoxyethyl cyclodextrin, sulfoxyl cyclodextrin, acetyl cyclodextrin, branched cyclodextrin, cyclodextrin fatty acid ester, glucosyl cyclodextrin, and maltosyl cyclodextrin. Preferably, α-cyclodextrin is used. Since α-cyclodextrin has a high solubility in water, an emulsified composition for skin with less roughness can be obtained when applied to the skin.

In the emulsified composition for skin of the present invention, the cyclodextrin may be contained in an amount capable of imparting emulsion stability to an oil-in-water emulsion together with a prescribed water-soluble gelling agent and providing a favorable feeling of use when the emulsified composition is applied to the skin. For example, the emulsified composition for skin of the present invention may contain cyclodextrin in an amount of 1 wt% or more or 2 wt% or more. The upper limit thereof is not particularly limited, but may be, for example, 15 wt% or less, 14 wt% or less, 13 wt% or less, or 12 wt% or less. The emulsified composition for skin of the present invention may contain cyclodextrin in an amount appropriately selected from the range of, for example, 1 wt% to 15 wt% or 2 wt% to 12 wt%. The emulsified composition for skin of the present invention may contain cyclodextrin in an amount greater than 3 wt%, for example, in an amount appropriately selected from the range of, 4 wt% or more, or 5 wt% or more, and 8 wt% or less, 7 wt% or less, or 6 wt% or less. If the amount of cyclodextrin is less than 1 wt%, the emulsion stability of the composition may be insufficient. On the other hand, if the amount of cyclodextrin is more than 15 wt%, the composition may have too high viscosity or give a strong creaky feeling when applied to the skin. In either case, the composition may not provide a favorable feeling of use when applied to the skin.

In the present invention, the "water-soluble gelling agent" generally means a substance that dissolves in water and imparts viscosity (sometimes also referred to as a thickener, a thickening stabilizer, or an adhesive, for example). Examples of the water-soluble gelling agent available in the present invention include carboxymethyl cellulose (CMC), xanthan gum, locust bean gum, guar gum, glucomannan, κ-carrageenan, t-carrageenan, λ-carrageenan, tamarind gum, and gellan gum. Each of the water-soluble gelling agents may be used alone or in combination with different water-soluble gelling agents. More preferably, the water-soluble gelling agents are carboxymethyl cellulose (CMC), glucomannan, tamarind gum, and xanthan gum, which can impart particularly high emulsion stability to the emulsified composition for skin of the present invention.

In the emulsified composition for skin of the present invention, the water-soluble gelling agent may be contained in an amount capable of imparting emulsion stability to an oil-in-water emulsion together with cyclodextrin and providing a favorable feeling of use when the emulsified composition is applied to the skin. For example, the emulsified composition for skin of the present invention may contain the water-soluble gelling agent in an amount of 0.1 wt% or more, 0.2 wt% or more, or 0.3 wt% or more. The upper limit thereof is not particularly limited, but may be, for example, 1 wt% or less, 0.8 wt% or less, 0.7 wt% or less, 0.6 wt% or less, or 0.5 wt% or less. The emulsified composition for skin of the present invention may contain the water-soluble gelling agent in an amount appropriately selected from the range of, for example, 0.1 wt% to 1 wt%, preferably 0.2 wt% to 0.8 wt%, and more preferably 0.3 wt% to 0.5 wt%. If the amount of the water-soluble gelling agent is less than 0.1 wt%, the emulsion stability of the composition may be insufficient. On the other hand, if the amount of the water-soluble gelling agent is more than 1 wt%, the composition may have too high viscosity. In either case, the composition may not provide a favorable feeling of use when applied to the skin.

Since the emulsified composition for skin of the present invention can impart high emulsion stability to the oil-in-water emulsion by the combined use of the cyclodextrin and the water-soluble gelling agent, the emulsified composition may be substantially free of a synthetic surfactant and/or a polyoxyethylene glycol, each of which is generally used as an emulsifier in a conventional emulsified composition for skin, in addition to the cyclodextrin and the water-soluble gelling agent. Preferably, the emulsified composition for skin of the present invention is substantially free of a synthetic surfactant and/or polyoxyethylene glycol. In the present invention, the phrase "substantially free of a synthetic surfactant and/or polyoxyethylene glycol" means that the emulsified composition for skin of the present invention does not contain a synthetic surfactant and/or polyoxyethylene glycol in such a manner as to exhibit an emulsifying effect, which is not intended to mean that the emulsified composition does not contain a synthetic surfactant and/or a polyoxyethylene glycol at all. The present invention can provide an emulsified composition for skin with high safety and less irritation to the skin by being substantially free of a synthetic surfactant. In addition, the emulsified composition for skin of the present invention can provide a smooth and moist feeling by washing after application because the emulsified composition is substantially free of polyoxyethylene glycol.

The emulsified composition for skin of the present invention has high emulsion stability that allows the emulsified state to be maintained even after treatment at high temperatures (e.g., about 100°C to 120°C). Accordingly, the emulsified composition for skin of the present invention can be subjected to a high-temperature sterilization treatment (e.g., retort sterilization treatment) in addition to/instead of the addition of an antiseptic, thus providing an emulsified composition for skin with low/no antiseptic content, high safety, and less irritation to the skin.

The emulsified composition for skin of the present invention has high emulsion stability with no/almost no oiliness such as stickiness or sliminess and a creaky feeling when applied to the skin. With no/almost no stickiness or sliminess when washed with water or the like after application, the emulsified composition also has a good, unique feeling of use, which simultaneously provides a smooth feeling as if a powder is applied and a moist feeling as if a cream is applied. As used herein, "washing" can be performed by a general method using water or hot water, and if necessary, a hand soap, a body soap, a facial cleansing foam, a soap, or the like.

The emulsified composition for skin of the present invention is not limited to any particular form and may be in a form of a gel or a dried body (e.g., a powder, or a flake) prepared by reducing the water content, in addition to a form having the above-described water content. The water content of the emulsified composition can be reduced by employing a conventionally known drying method. Examples of such a drying method include, but are not limited to, freeze drying, heat drying, air drying, spray drying, drum drying, hot air drying, and vacuum drying. The water content of the emulsified composition after drying can be appropriately selected depending on the intended form, and may be, for example, less than 15 wt%, 10 wt% or less, 5 wt% or less, or 1 wt% or less. The lower limit of the water content of the emulsified composition after drying is not particularly limited but can be preferably 0.1 wt% or more, 0.5 wt% or more, 0.6 wt% or more, 0.7 wt% or more, or 0.8 wt% or more. The water content of the emulsified composition after drying may be, for example, 0.1 wt% to less than 15 wt%, 0.1 wt% to 10 wt%, 0.5 wt% to 5 wt%, 0.5 wt% to 1 wt%, 0.6 wt% to 1 wt%, 0.7 wt% to 1 wt%, or 0.8 wt% to 1 wt%. The emulsified composition for skin of the present invention, even in the form of a gel or a dried body, can be used in the same manner as an emulsified composition that has not been subjected to a drying treatment. The emulsified composition has a good, unique feeling of use, in which the skin is coated in a transparent state such that an oil film is quickly formed with no/almost no oiliness such as stickiness or sliminess and a creaky feeling when spread on the skin while a smooth feeling as if a powder is applied and a moist feeling as if a cream is applied are simultaneously provided with no/almost no stickiness or sliminess when washed with water or the like after application.

In addition to the above-described components, the emulsified composition for skin of the present invention may further contain, if necessary, components usually used in the production of cosmetics and pharmaceuticals (including quasi-drugs), as long as the effects of the present invention are not impaired. Examples of such components include, but are not limited to, humectants (polyglycerin, hyaluronic acid, chondroitin sulfate, glycerin, sorbitol, xylitol, maltitol, erythritol, mucopolysaccharides, chitosan, 1,3-butylene glycol, lactic acid, sodium pyrrolidonecarboxylate, urea, etc.), solvents (ethanol, methanol, isopropyl alcohol, acetone, hexane, ethyl acetate, isopropyl acetate, butyl acetate, isobutyl acetate, chloroform, etc.), organic acids (citric acid, malic acid, tartaric acid, lactic acid, glutathione, etc.), vitamins (vitamin A, vitamin B, ascorbic acid, vitamin C, vitamin D, vitamin H, pantothenic acid, etc.), drugs, plant extracts, inorganic pigments (talc, mica, titanated mica, yellow iron oxide, titanium oxide, zinc white, zinc oxide, kaolin, calcium carbonate, ultramarine, bentonite, carbon black, black iron oxide, silicic acid anhydride, red iron oxide, light calcium carbonate, heavy calcium carbonate, light magnesium carbonate, heavy magnesium carbonate, etc.), perfumes, ultraviolet absorbers, excipients, preservatives, antiseptics, antioxidants, nonionic surfactants, cationic surfactants, anionic surfactants, amphoteric surfactants, chelating agents, and pH adjusters.

The emulsified composition for skin of the present invention can be used as a base for cosmetics and pharmaceuticals (including quasi-drugs) that are applied to the skin and provided in any form including, but not limited to, topical medications (ointments, creams, and lotions), hand creams, skin care creams, body creams, milky lotions, cleansing creams, after-shaving creams, cold creams, shaving creams, burnishing creams, sunburn creams, sunscreen creams, basic cosmetics, creamy foundations, makeup cosmetics, and lip balms. The emulsified composition for skin of the present invention is preferably provided in the form of hand cream. In particular, hand creams using edible vegetable oils as fat and oil are safe even when put in the mouth, thus being used without anxiety in a kitchen, a kitchen counter, or the like. Note that the emulsified composition for skin of the present invention may not have a cream-like form in the case of being in the form of a gel or a dried body obtained by the drying treatment, but can coat the skin in a transparent state such that an oil film is quickly formed when spread on the skin even in the form of a gel or a dried body. Therefore, the emulsified composition can be provided in any form such as the above-described topical medications (ointments, creams, and lotions) and hand creams as in the case of an emulsified composition that has not been subjected to a drying treatment.

The emulsified composition for skin of the present invention can be produced by mixing and stirring the water, fat and oil, cyclodextrin, and the prescribed water-soluble gelling agent, as well as other components, if necessary, in the above-described amounts. All of the components may be mixed and stirred together, or each component may be added separately or sequentially in any combination (in any order), then mixed and stirred. The emulsified composition obtained by mixing and stirring may be subjected to a heat sterilization treatment. The emulsified composition obtained by mixing and stirring may be further subjected to a drying treatment, if necessary. The drying treatment can be performed by the conventionally known drying method, and the water content of the emulsified composition can be appropriately adjusted according to the intended form. The emulsified composition subjected to the drying treatment may be further subjected to crushing, grinding, or abrasion treatment, if necessary, and other components may be mixed and stirred in the above-described amounts, if necessary.

Hereinafter, the present invention will be described with reference to Examples, but the present invention is not limited to these Examples.

### Examples

### Experiment 1: Evaluation of Emulsion Stability

### (1) Preparation of emulsified composition

According to the composition in Table 1 below, each component was added and mixed to prepare an emulsified composition. As the water-soluble gelling agent, any one of carboxymethyl cellulose (CMC), sodium alginate, gum arabic, glucomannan, guar gum, pectin, κ-carrageenan, tamarind gum, gellan gum, xanthan gum, t-carrageenan, locust bean gum, λ-carrageenan, distarch phosphate, hydroxypropyl starch, hydroxypropylated distarch phosphate, tragacanth gum, carboxyvinyl polymer, sodium polyacrylate, hydroxypropyl cellulose, methyl cellulose, hydroxypropylmethyl cellulose, and hydroxyethyl cellulose was blended.

Each component was mixed and stirred using a hand blender at 20,000 rpm for 10 minutes. Each composition (50 mL) obtained was transferred to a conical tube (Falcon (R) Conical Tube 50 mL) and centrifuged at 3,000 rpm for 1 minute. Subsequently, the thicknesses (depths) of water phase, micelles, and oil phase were visually observed, and the proportion of each phase (%) was measured, thereby evaluating the emulsion stability of each composition. The evaluation was made as "⊚" when the proportion of micelles was 100%, as "○" when the proportion of micelles was 70% or more and less than 100%, and as "×" when the proportion of micelles was less than 70%.

The amount of each component in the table is expressed as wt%. All of the fat and oil, α-cyclodextrin, and water-soluble gelling agents used were food additives (food grade).

**[Table 1]**

| Component | Blending amount (wt%) |
|---|---|
| Fat and oil (Canola oil + 1% oil red) | 25 |
| α-cyclodextrin | 2.5 |
| Water-soluble gelling agent | 0.35 |
| Water | 72.15 |
| Total | 100 |

### (2) Result

The water-soluble gelling agent added to the emulsified composition, the measured proportion of each phase, and the evaluation results are shown in Table 2. As is clear from the results, the emulsion stability of the emulsified composition varied depending on the water-soluble gelling agent added. When CMC, glucomannan, guar gum, κ-carrageenan, tamarind gum, gellan gum, xanthan gum, t-carrageenan, locust bean gum, or λ-carrageenan was added, the micelle phase was maintained and the emulsified composition exhibited high emulsion stability. In particular, when CMC, glucomannan, tamarind gum, or xanthan gum was added, separation of the water phase and the oil phase was not observed while remarkably high emulsion stability was confirmed.

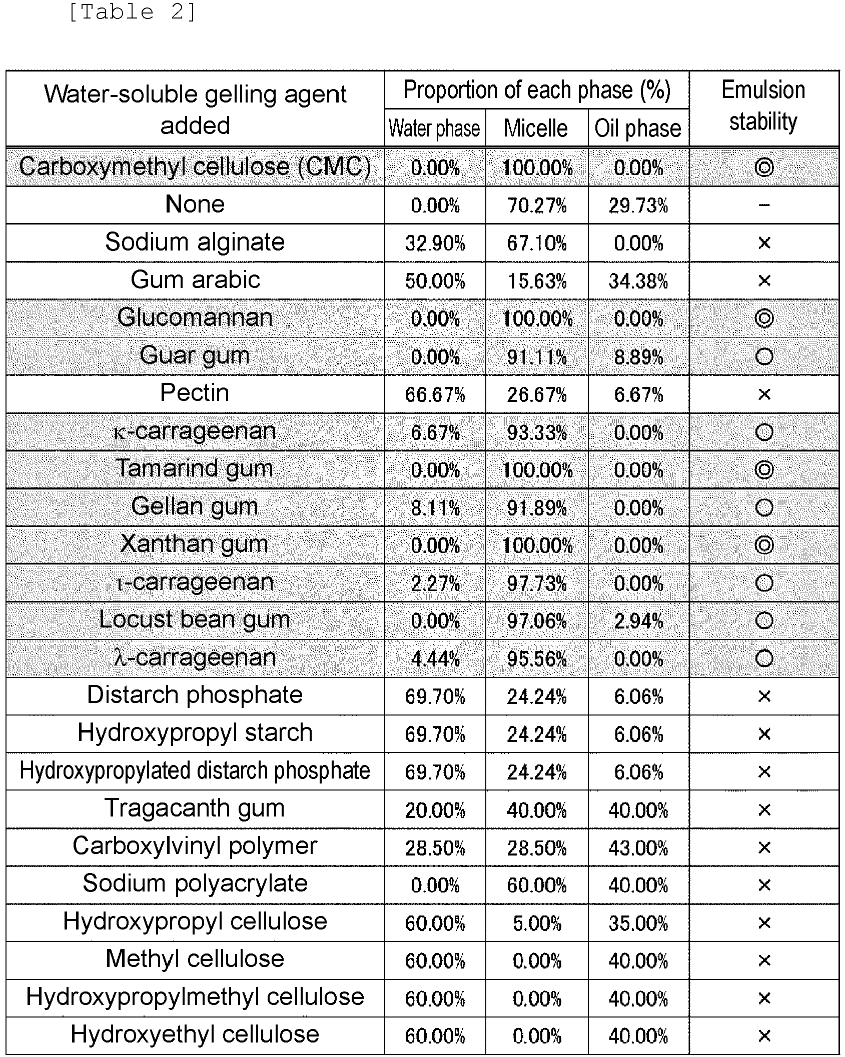

### Experiment 2: Evaluation of Interaction between α-Cyclodextrin and Water-Soluble Gelling Agent

To evaluate the interaction between α-cyclodextrin and a water-soluble gelling agent in an aqueous solution, enthalpy change (ΔH) was determined by isothermal titration calorimetry (ITC) in accordance with a conventionally known method. Specifically, using an isothermal titration calorimeter (NANO ITC SV; TA Instruments), 10 µL of an aqueous solution of water-soluble gelling agent (0.05 g/100 mL) was added dropwise to an aqueous solution of α-cyclodextrin (25 g/100 mL) 25 times at intervals of 180 seconds (75 minutes) to determine an enthalpy value (µJ) from the area of the titration peak at the 25th (final) titration.

The results are shown in Table 3. Positive enthalpy values, i.e., endothermic reactions, were observed only when CMC and xanthan gum, which had high emulsion stability in Experiment 1, were used. This result suggests the presence of an interaction between the water-soluble gelling agent and α-cyclodextrin via hydration water, and it is considered that, for example, a part of water molecules hydrated in the water-soluble gelling agent forms a hydrogen bond with α-cyclodextrin. A hydrogen bond formed between water molecules usually has a shorter distance and a higher energy value than those of a hydrogen bond formed between the water-soluble gelling agent and α-cyclodextrin, which causes intermolecular repulsion due to a charged site. Therefore, when a part of water molecules hydrated in the water-soluble gelling agent forms a hydrogen bond with α-cyclodextrin, it is considered that an endothermic reaction occurs as a result of the subtraction of energy values. The heat transfer (positive enthalpy value) suggests the presence of the interaction between the water-soluble gelling agent and α-cyclodextrin, and this interaction is considered to be a factor in giving high emulsion stability in Experiment 1.

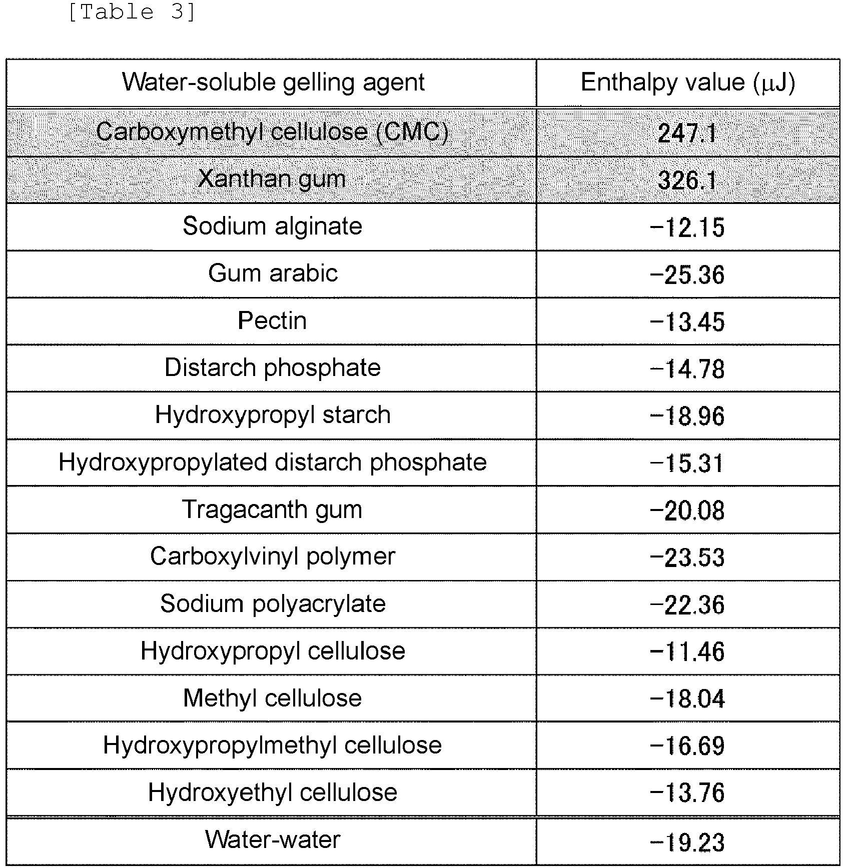

### Experiment 3: Performance Evaluation

### (1) Preparation of emulsified composition

According to the composition in Table 4 below, each component was added and mixed to prepare emulsified compositions of Examples 1 to 6 and Comparative Examples 1 to 4. Each component was mixed and stirred using a hand blender at 20,000 rpm for 10 minutes. Each emulsified composition obtained was evaluated for the following evaluation items based on the criteria. The evaluation was made on four human subjects.

Creaminess: when the emulsified compositions are each spread on the palm of the subject, "○" in the case of having a good feeling of use (viscosity) as a hand cream; "△" in the case of having a viscosity usable as a hand cream; and "×" in the case of being unsuitable for use as a hand cream (too low viscosity or too high viscosity).

Emulsifying ability after storage: "○" in the case where emulsification is maintained after storage at 40°C for 7 days; "×" in the case where separation of water and oil is observed.

Slimy feeling immediately after application: immediately after application to the palm of the subject, "○" in the case of the absence of oiliness (stickiness or sliminess); and "×" in the case of the presence of oiliness.

Feeling of use after washing: when the emulsified compositions are each applied to the palm of the subject and then washed, "○" in the case where a smooth and moist feeling is recognized, "△" in the case where a smooth and moist feeling is recognized with slight oiliness at the same time, and "×" in the case where a smooth and moist feeling is not recognized. Although the same result can be obtained when only water is used or when hand soap is used for washing, the hand soap was used for washing in this experiment.

Heat sterilization: "○" in the case where emulsification is maintained after retort sterilization (123°C, 50 minutes), "×" in the case where separation of water and oil is observed.

The amount of each component in the following table is expressed as wt%.

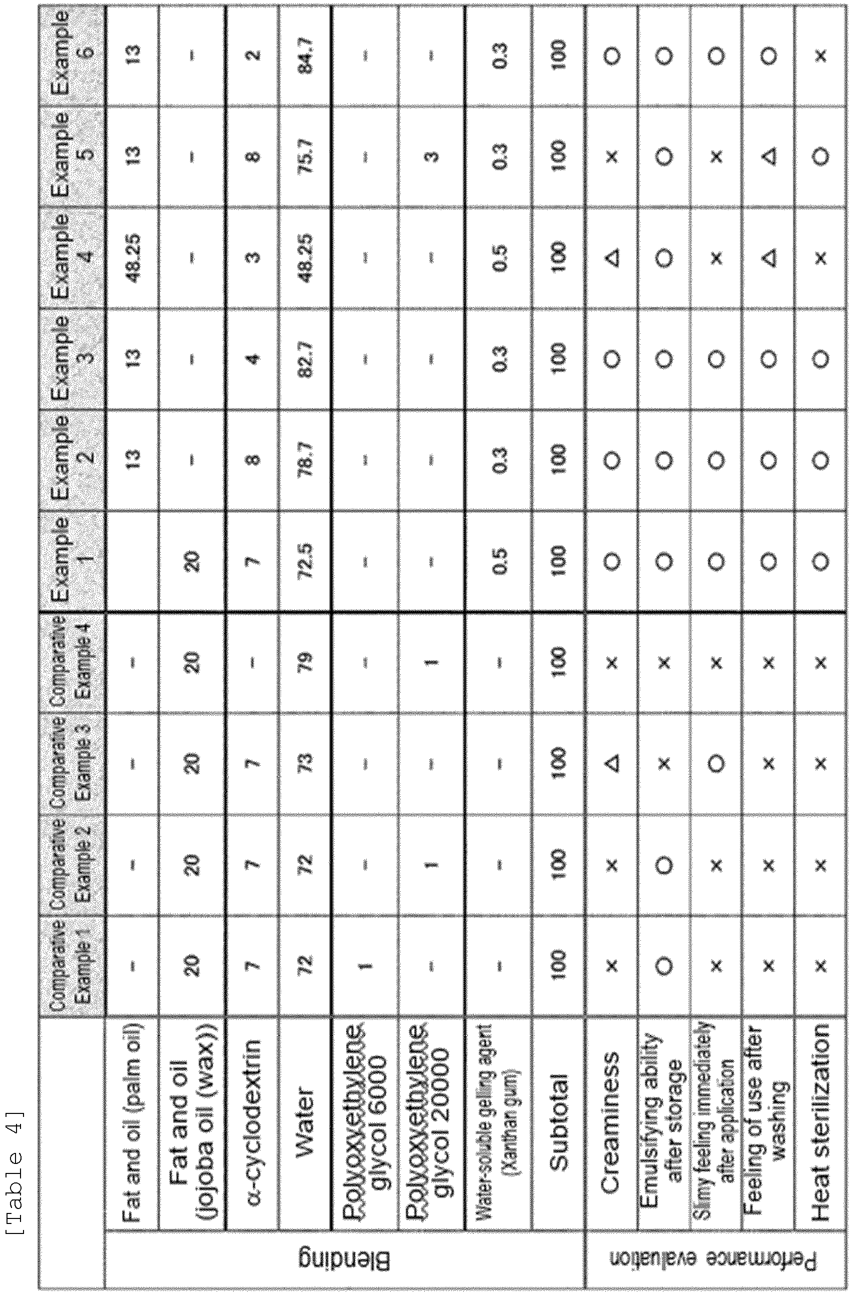

### (2) Result

The results of each evaluation for each emulsified composition obtained are shown in Table 4.

From the results of Examples 1 to 6, it was confirmed that the fat and oil is not limited to specific ones, and various fat and oil can be used.

When polyoxyethylene glycol was used instead of the water-soluble gelling agent (Comparative Examples 1, 2, and 4), some of the following were observed: creaminess unsuitable for use as a hand cream, a decrease in emulsifying ability after storage, a decrease in the feeling of use immediately after application and after washing, and a decrease in emulsifying ability after heat sterilization, and it was confirmed that the performance was inferior to that of the emulsified compositions of Examples 1 to 6 using the water-soluble gelling agent.

Although the emulsified compositions of Examples had performances that could be used as a hand cream, the emulsified compositions of Examples 1 to 3 had particularly excellent performance and showed good results in all evaluation items. In Examples 1 to 3, Examples 1 and 2 in which the content of α-cyclodextrin was relatively high gave a slight feeling of tightness (creaky feeling) (not to such an extent as to cause a problem as a hand cream) when applied as compared with Example 3 in which the content of α-cyclodextrin was relatively low.

Experiment 4: Powder Emulsified Composition (Freeze-Dried)

### (1) Preparation of powder emulsified composition

According to the composition in Table 5 below, each component was added and mixed to prepare an emulsified composition. Each component was mixed and stirred using a hand blender at 20,000 rpm for 5 minutes. Next, the resulting emulsified composition was freeze-dried (at 20°C for 16 hours) and crushed to obtain a powder emulsified composition.

**[Table 5]**

| Component | Blending amount (wt%) |
|---|---|
| Fat and oil (palm oil) | 13 |
| α-cyclodextrin | 8 |
| Water-soluble gelling agent (CMC) | 0.3 |
| Water | 78.7 |
| Total | 100 |

### (2) Performance evaluation of powder emulsified composition

(i) The water content of the obtained powder emulsified composition was measured by a normal pressure heating and drying method in accordance with an ordinary method. Specifically, the obtained emulsified composition was put in a weighing can and precisely weighed (weight before drying), dried at 105°C for 5 hours, then transferred to a desiccator, allowed to cool for 1 hour, and precisely weighed (weight after drying). The water content (wt%) of the powder emulsified composition was determined from the weight before drying and the weight after drying.
(ii) The obtained powder emulsified composition was evaluated for "creaminess", "emulsification ability after storage", "slimy feeling immediately after application", and "feeling of use after washing" by the same method and criteria as those described in "Experiment 3: Performance Evaluation". As a control, a composition obtained by the same operation was used, except that each component was added according to the composition in Table 5 and mixed by hand without applying a shear force (i.e., an emulsified composition was not prepared) to prepare the composition.

### (3) Result

(i) The measurement results of water content are shown in Table 6. The water content was measured using two powder emulsified compositions individually prepared as samples. The water contents of the obtained powder emulsified compositions were about 0.7 wt% and about 0.9 wt%, and the average was about 0.8 wt%.

**[Table 6]**

| Sample | Weighing can weight (g) | Weight before drying (g) | Weight after drying (g) | Water content (wt%) (Average value) | |
|---|---|---|---|---|---|
| No.1 | 16.4025 | 16.9279 | 16.924 | 0.742 | 0.807 |
| No.2 | 17.0752 | 17.9472 | 17.9396 | 0.872 | |

| | | | | | |
|---|---|---|---|---|---|
| The weight before drying and the weight after drying indicate the total weight of the weighing can and the powder emulsified composition. | | | | | |

(ii) The powder emulsified composition had a form of a free-flowing powder, whereas the control composition had a form of a paste in which fat and oil as liquids and a powder were mixed together, and thus a form of a free-flowing powder was not obtained.

The obtained powder emulsified composition was evaluated as "○" in all of "creaminess", "emulsification ability after storage", "slimy feeling immediately after application", and "feeling of use after washing". Similarly to the emulsified composition of Experiment 3, the powder emulsified composition had a good feeling of use as a hand cream, in which the skin was coated in a transparent state such that an oil film was quickly formed when spread on the palm of the subject. The powder emulsified composition also simultaneously provided a smooth feeling as if a powder was applied and a moist feeling as if a cream was applied when washed after application. On the other hand, the control composition did not provide a good feeling of use when applied to the palm of the subject because the liquid and the solid were separated from each other, giving a rough and creaky feeling.

### Experiment 5: Powder Emulsified Composition (Spray-Dried)

### (1) Preparation of powder emulsified composition

According to the composition in Table 5, each component was added and mixed to prepare an emulsified composition. Each component was mixed and stirred using a hand blender at 20,000 rpm for 5 minutes. Next, the resulting emulsified composition was spray-dried (190°C) to obtain a powder emulsified composition.

### (2) Performance Evaluation of Powder Emulsified Composition

(i) The water content (wt%) of the obtained powder emulsified composition was determined in the same manner as described in "Experiment 4: Powder Emulsified Composition (Freeze-dried)".
(ii) The obtained powder emulsified composition was evaluated for "creaminess", "emulsification ability after storage", "slimy feeling immediately after application", and "feeling of use after washing" in the same manner as described in "Experiment 4: Powder Emulsified Composition (Freeze-dried)".

### (3) Result

(i) The measurement results of water content are shown in Table 7. The water content was measured using two powder emulsified compositions individually prepared as samples. The water contents of the obtained powder emulsified compositions were about 0.7 wt% and about 1.0 wt%, and the average was about 0.8 wt%.

**[Table 7]**

| Sample | Weighing can weight (g) | Weight before drying (g) | Weight after drying (g) | Water content (wt%) (Average value) | |
|---|---|---|---|---|---|
| No.1 | 16.125 | 18.2926 | 18.2717 | 0.964 | 0.809 |
| No.2 | 16.9779 | 18.923 | 18.9103 | 0.653 | |

| | | | | | |
|---|---|---|---|---|---|
| The weight before drying and the weight after drying indicate the total weight of the weighing can and the powder emulsified composition. | | | | | |

(ii) Similarly to the freeze-dried composition, the spray-dried powder emulsified composition also had a form of free-flowing powder and was evaluated as "○" in all of "creaminess", "emulsification ability after storage", "slimy feeling immediately after application", and "feeling of use after washing". The spray-dried powder emulsified composition had a good feeling of use as a hand cream, in which the skin was coated in a transparent state such that an oil film was quickly formed when spread on the palm of the subject. The powder emulsified composition also simultaneously provided a smooth feeling as if a powder was applied and a moist feeling as if a cream was applied when washed after application. It was confirmed that even when the drying treatment was performed in a different manner, the same characteristics were obtained.

The results revealed that an emulsified composition having high emulsion stability and performance suitable for application to the skin can be obtained by blending cyclodextrin and a prescribed water-soluble gelling agent in combination with an oil-in-water emulsion containing water and fat and oil. It has been confirmed that when the emulsified composition is spread on the skin, the feeling of use is unprecedented, and the emulsified composition has a good feeling of use as a hand cream and simultaneously provided a smooth feeling as if a powder was applied and a moist feeling as if a cream was applied when washed with water or the like after application.

Further, it was confirmed that a dried form (powder emulsified composition) obtained by subjecting the emulsified composition to a drying treatment also coats the skin in a transparent state such that an oil film is quickly formed when spread on the skin, and thus provides the same characteristics. On the other hand, these characteristics were not obtained for the dried form obtained by subjecting the emulsified composition to a drying treatment without passing through the emulsified composition having the high emulsion stability. Therefore, the good characteristics in dried form are also due to the maintenance of the emulsified state without solid-liquid separation, which is attributed to the high emulsion stability of the emulsified composition before being subjected to a drying treatment.

## Claims

1. An emulsified composition for skin, comprising water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum.

2. The emulsified composition according to claim 1, wherein the fat and oil is an edible vegetable oil.

3. The emulsified composition according to claim 1 or 2, which is a hand cream.

4. A method for producing an emulsified composition for skin, comprising mixing water, fat and oil, cyclodextrin, and at least one water-soluble gelling agent selected from the group consisting of carboxymethyl cellulose (CMC), xanthan gum, locust bean gum, guar gum, glucomannan, κ-carrageenan, t-carrageenan, λ-carrageenan, tamarind gum, and gellan gum together.

5. The method according to claim 4, wherein the fat and oil is an edible vegetable oil.

6. The method according to claim 4 or 5, wherein the emulsified composition for skin is a hand cream.
